# EUROPEAN PATENT SPECIFICATION

(11) **EP 2 402 053 B1**
(45) Date of publication and mention of the grant of the patent: **26.03.2014**
(21) Application number: 10745800.2
(22) Date of filing: 15.01.2010
(51) Int. Cl.: A61N 1/20, A61B 18/00, A61B 18/14

(54) **ELECTROHYDRAULIC PROSTATE TISSUE TREATMENT PROBE**
ELEKTROHYDRAULISCHE SONDE ZUR BEHANDLUNG VON PROSTATAGEWEBE
SONDE ÉLECTRO-HYDRAULIQUE DE TRAITEMENT DU TISSU PROSTATIQUE

(30) Priority: 24.02.2009 CN 200910010438
(43) Date of publication of application: 04.01.2012
(73) Proprietor: Jiang, Qijun, Liaoning 110015 (CN)
(72) Inventor: Jiang, Qijun, Liaoning 110015 (CN)
(74) Representative: Hervius Löthman, Johan
(86) International application number: PCT/CN2010/070195
(87) International publication number: WO 2010/097016

(56) References cited:
- CN-A- 1 189 382
- CN-A- 1 586 663
- CN-A- 101 485 592
- CN-Y- 2 669 899
- CN-Y- 201 006 054
- CN-Y- 201 006 054
- DE-A1-102007 010 075
- DE-A1-102007 010 075
- US-A- 5 249 585
- US-A- 5 249 585
- US-A- 5 529 574

## Description

### Field of the invention

The present disclosure relates to an electrolytic treatment probe, in particular a probe for treating prostate tissue by electrolytic liquefying, which is a preferred treatment probe for the treatment of hyperplasia of prostate using prostate electrolytic therapeutic apparatus in various medical institutions.

The invention is set out in the appended claims.

### Background of the invention

Prostatic hyperplasia is a common disease of older men, which is a disease characterized by progressive dysuria as its main clinical symptom. In the early stage, frequent micturition, urgent urination, frequent nocturia, and the like are the main irritative symptoms. After developing into bladder outlet obstruction, clinical manifestations are symptoms of waiting for the beginning of the urine, urine flow thinning, urine flow interruption, post-micturition dribble and feeling of unable to complete urination. After the bladder detrusor loses its compensatory abilities, life-threatening severe complications, e.g. overflow incontinence, hydronephrosis, renal failure, and the like may also appear. And often emergencies such as abdominal hernia, urinary system lithiasis, urinary system infection, bladder hematorrhea, urinary retention, and the like can be caused. At present, there is still no drug that can inhibit the pathological process of prostatic hyperplasia, avoid bladder outlet obstruction and prevent urinary retention. Transurethral prostatic resection is considered worldwide as the "gold standard" for the treatment of prostatic hyperplasia. In recent years, many hospitals in our country have been carrying out this type of surgery. However, at present, due to the great complexity of such surgical operation technology, professional doctors who can proficiently carry out such surgeries independently in all provinces, cities and regions are still very few. In particular, for the elderly patients who can no longer undergo anesthesia due to their physical weakness, so as to lose the opportunity for surgical treatment, they can only address the problem of urination by receiving fistulation or repeated urethral catheterizations. Accordingly, Professor JIANG Qijun, a urology scholar in Chinese, successfully developed "prostate electrolytic treatment probe", which was filed for invention patent on Jan. 19th, 1993, and had been patented with the patent number ZL93110056.9. It is a urethral interventional therapy for prostatic hyperplasia. During 15 years of clinical application, it had treated more than one million cases of prostatic hyperplasia, with a clinical effective rate over 87%, and no death case was reported in such treatment or around such treatment period, according to the feedback information of clinical application from over 300 domestic medical institutions. Up to now, it is the safest method for the treatment of prostate diseases.

However, through nearly a hundred cases of in vitro and in vivo pathological examination studies, it has been found that there are still unsatisfactory defects for this prostate electrolytic treatment probe which need to be improved. Such defects are as follow: 1. the radial therapeutic range of liquefied tissue is small, with its maximum radius of 10mm, so that for the patients with larger prostatic hyperplasia, obstructions often occur again in two years after the cure; 2. in the later stage of the therapeutic process, the drainage pipe is prone to clog due to a lot of shedding tissue scraps, while the anode electrode in the pipe is surrounded by gas film, so that the phenomena of therapeutic current interruptions often occur; 3. electrode in the anode electrode is made of expensive platinum-rhodium alloy wire, and it must be strictly disinfected for repeated uses, otherwise, risks of cross infection would be presented.

### Summary of the invention

In view of the problems existing in the prior art, the present invention tends to provide a probe for treating prostate tissue by electrolytic liquefying, which is characterized by a larger radial therapeutic range, faster recovery after thorough treatment, significant improvement of therapeutic effects without relapse after the cure, stable therapeutic current unaffected by the blockage of drainage pipe, prevention of cross infection and lower costs.

Another purpose of the present invention is to provide a safe and effective non-surgical method for the urethral interventional electrolytic liquefying therapy of prostatic hyperplasia, which is characterized by easy outpatient treatment, surface anesthesia of urethra, simple operation, well tolerated by patients, mild adverse reaction, which is especially suitable for the prostatic hyperplasia treatment of elderly high-risk patients who can not tolerate surgical therapy, and for the early stage patients. The electrolytic liquefying treatment of prostatic hyperplasia using the probe for treating prostate tissue by electrolytic liquefying is a safe, effective and clinically practical therapeutic technology, which can be applied in both medium and small hospitals.

The present invention is realized by the following technical solutions:
The probe comprises a probe pipe body, in which a drainage channel, a water-injection channel, a gas-injection channel and a cathode channel are arranged along the axial direction. The front end of the probe pipe body is a guiding head, behind which a positioning balloon is arranged. An external cathode electrode is wound tightly around the probe pipe body on the rear side of the positioning balloon (that is, on the opposite side of the positioning balloon to the side of the guiding head), and the leading-out end of the external cathode electrode is connected with a cathode plug through the cathode channel. An external anode electrode is arranged between the positioning balloon and the cathode electrode, and is wound around the probe pipe body by at least one circle. The external anode electrode is kept at a distance from the However, through nearly a hundred cases of in vitro and in vivo pathological examination studies, it has been found that there are still unsatisfactory defects for this prostate electrolytic treatment probe which need to be improved. Such defects are as follow: 1. the radial therapeutic range of liquefied tissue is small, with its maximum radius of 10mm, so that for the patients with larger prostatic hyperplasia, obstructions often occur again in two years after the cure; 2. in the later stage of the therapeutic process, the drainage pipe is prone to clog due to a lot of shedding tissue scraps, while the anode electrode in the pipe is surrounded by gas film, so that the phenomena of therapeutic current interruptions often occur; 3. electrode in the anode electrode is made of expensive platinum-rhodium alloy wire, and it must be strictly disinfected for repeated uses, otherwise, risks of cross infection would be presented.

CN-A-201006054 discloses a disposable electrode catheter for treating prostrate, urethra and bladder diseases. It comprises a tube, a front positioning air-bag, a back positioning air-bag, a front positioning air-bag gas tube, a back positioning air-bag gas tube, a medicine-filling device, a drainage pipe and a plug. The front positioning air-bag gas tube and the back positioning air-bag gas tube are composed of a one-way valve, a detecting air-bag and a siphonium. The medicine-filling device is composed of a medicine filling valve and a siphonium, and the drainage device is composed of a round table and a drainage pipe.

### Summary of the invention

In view of the problems existing in the prior art, the present invention tends to provide a probe for treating prostate tissue by electrolytic liquefying, which is characterized by a larger radial therapeutic range, faster recovery after thorough treatment, significant improvement of therapeutic effects without relapse after the cure, stable therapeutic current unaffected by the blockage of drainage pipe, prevention of cross infection and lower costs.

Another purpose of the present invention is to provide a safe and effective non-surgical method for the urethral interventional electrolytic liquefying therapy of prostatic hyperplasia, which is characterized by easy outpatient treatment, surface anesthesia of urethra, simple operation, well tolerated by patients, mild adverse reaction, which is especially suitable for the prostatic hyperplasia treatment of elderly high-risk patients who can not tolerate surgical therapy, and for the early stage patients. The electrolytic liquefying treatment of prostatic hyperplasia using the probe for treating prostate tissue by electrolytic liquefying is a safe, effective and clinically practical therapeutic technology, which can be applied in both medium and small hospitals.

The present invention is realized by the following technical solutions:
The probe comprises a probe pipe body, in which a drainage channel, a water-injection channel, a gas-injection channel and a cathode channel are arranged along the axial direction. The front end of the probe pipe body is a guiding head, behind which a positioning balloon is arranged. An external cathode electrode is wound tightly around the probe pipe body on the rear side of the positioning balloon (that is, on the opposite side of the positioning balloon to the side of the guiding head), and the leading-out end of the external cathode electrode is connected with a cathode plug through the cathode channel. An external anode electrode is arranged between the positioning balloon and the cathode electrode, and is wound around the probe pipe body by at least one circle. The external anode electrode is kept at a distance from the the liquefied necrotic tissues shed and are discharged by flushing without any adhesion to the electrode, electrolytic liquefied necrotic tissues all shed completely in one month after the treatment, and less recurrence, and basal wound can be repaired by urothelium mucosa, less producing fibroplasia and cicatricial constriction; even if the drainage pipe is blocked by tissue shedding during the treatment, therapeutic current can still be maintained within the set range, without affecting the smooth conduct of the treatment. In addition, the present invention can significantly reduce the costs of probe materials, in which the amount of platinum-rhodium alloy electrode used is 5% of that in the prior art, and the risks for cross infections are effectively eliminated, the patient's tolerance to it is good, the adverse reactions are mild, so that it is suitable for the treatment of prostatic hyperplasia patients of various stages in various types of hospitals, in particular more suitable for the treatment of elderly high-risk prostatic hyperplasia patients who can not tolerate surgery and anesthesia.

### Description of the drawings

Figure 1 is a schematic diagram of structure of a probe for treating prostate tissue by electrolytic liquefying according to the present invention.
Figure 2 is a schematic cross section along A-A of Figure 1.

In the figures, 1 water-injection inlet, 2 drainage outlet, 3 gas-injection one-way valve, 4. bracing wire channel, 5. cathode channel, 6. anode electrode, 7. cathode electrode, 8. positioning balloon, 9. guiding head, 10. probe pipe body, 11. inlet of drainage liquid, 12. air outlet, 13. water outlet, 1-1. water-injection channel, 2-1. drainage channel, 3-1. gas-injection channel, A. cathode plug, B. anode plug.

### Detailed embodiments

### Examples

The present invention is described in detail by examples combined with figures.

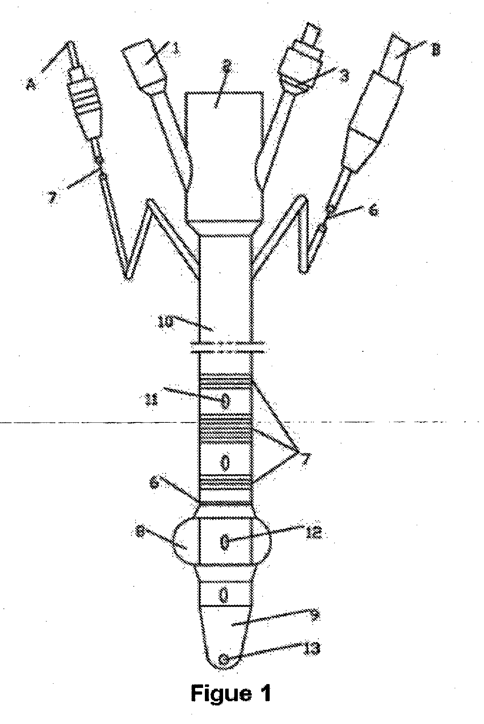

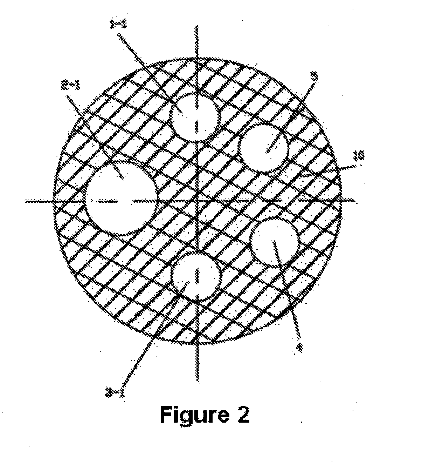

Figure 1 shows the schematic diagram of structure of a probe for treating prostate tissue by electrolytic liquefying according to the present invention. Probe pipe body 10 is made of medical silica gel. A guiding head 9 is disposed at the front end of the Probe pipe body 10, and the guiding head 9 has a water outlet 13 and an inlet of drainage liquid 11 on it. Behind the guiding head 9, a positioning balloon 8 is arranged on the probe pipe body 10, for example the positioning balloon 8 clasps the probe pipe body 10 as a sheath. There is an air outlet 12 arranged on the side walls of the positioning balloon 8 and the probe pipe body 10. On the rear end of probe pipe body 10, a cathode plug A, a water-injection inlet 1, a drainage inlet 2, a gas-injection one-way valve 3 and an anode plug B are arranged which are connected to their corresponding channels. One end of said cathode plug A is connected to the cathode electrode 7 by a medical stainless steel alloy wire with a diameter of less than 0.3mm through cathode channel 5, the drainage outlet 2 is communicated with the inlet of drainage liquid 11 through the drainage channel 2-1, the water-injection inlet 1 is communicated with the water outlet 13 through the water-injection channel 1-1, the gas-injection one-way valve 3 is communicated with the air outlet 12 through the gas-injection channel 3-1, anode plug B is connected to the anode electrode 6, which is arranged between the positioning balloon 8 and the cathode electrode 7, by a platinum-rhodium alloy wire with a diameter of 0.3mm through the drainage channel 2-1. The anode electrode 6 is wound around probe pipe body 10 by 1-3 circles, and is arranged between the positioning balloon 8 and the cathode electrode 7 while closer to the side of positioning balloon 8 and with a spacing of 3-5mm to cathode electrode 7. The cathode electrode 7 is wound around the probe pipe body 10 along the axial direction with intervening spaces, and is composed of several segments; within one segment, the cathode electrode is wound tightly, while between the segments, the spacing is 5mm (see the numeral sign 7 of Fig.1); and each segment can has different length, for example the length of one of the segments can be 5-10mm, and the length of each of the others is 5mm (the segment length means the length of one segment along the axial direction after single-layer winding tightly). On the pipe wall of the probe between each segment of electrode, inlets of drainage liquid are arranged, which are communicated with the drainage channel 2-1. A bracing wire channel 4, which prevents the tensile deformation of pipe body, is arranged within the effective length range in said probe pipe body 10 along the axial direction, an anti-stretch flexible wire is placed in the bracing wire channel 4, with both its ends fixed and connected to the pipe body 10, as is shown in Figure 2, which can effectively prevent disorders of therapeutic electrodes caused by the tensile deformation of the pipe body, so as to guarantee the accurate positioning of the therapeutic electrodes.

Applied examples: Zhang, male, 88-year-old, has been diagnosed to be a patient with prostatic hyperplasia complicated by urinary retention. According to the length of his prostatic urethra, which is 58mm, an appropriate treatment probe of 50mm was chosen, and therapeutic quantity of electricity of 400 coulomb (C) was given, (using 200 C electrical quantity for 30mm electrode of the probe as a reference, electrical quantity increases 50 C for each additional 5mm of the electrode length). First, sending the probe into bladder through urethra according to the procedure of urethral catheterization, injecting 10ml water into the positioning balloon 8, stopping pulling out the probe after the pulling be hindered, fixing the treatment probe in such a state without pulling resistance. After completing all connections, emptying the residual urine, closing the drainage pipe, starting instilling 200ml normal saline into the bladder, opening the drainage pipe again which is connected to a urine collection bag, placing the urine collection bag on the location which is 100-200mm over the pubic bone in order to keep some of the flushing fluid stored in the bladder while draining unimpeded, controlling the perfusion drainage volume at 1000ml/hr. then turning on the device for the treatment, using a current of less than 30mA at first, after the accumulation of the electrical quantity reaches 50 C, gradually raising the therapeutic current to 80mA±10mA according to the status of the patient's tolerance, until reaching the therapeutic quantity of electricity. Maintaining a perfusion washing flow of 1000 ml/hr during the process of treatment, when therapeutic quality of electricity is completed, perfusing 200ml normal saline remained in the bladder, discharging the water in the positioning balloon, pulling out the treatment probe, and immediately putting in a balloon catheter (F16) for a urinal indwelling catheterlization for 24hr to 2 weeks.

12 patients, who were aged 57-88 years old with their prostate volume 35-67g, were clinically applied, all of these patients were complicated by urinary retention.

The patients who underwent such treatment were all receiving the treatment safely and stably under only surface anesthesia of urethra, no abnormalities or adverse reactions were observed; during the treatment, the current kept stable with fluctuation range being less than 5mA, and no phenomenon of power failure alarm due to impeded drainage occurred; in 24hrs to 2 weeks after the treatment, all the patients restored their abilities to urinate on their own; 3 weeks later, urinary flow rate detections were performed, the maximum urinary flow rates were all greater than 25 ml/s; observed for 1.5-2 years, all the patients urinated normally, the phenomena of urinary retention have not been found yet.

The in vitro and in vivo results were further verified by the results of clinical trials. The same animal muscle tissue was used, and the same therapeutic quantity of electricity was given. Comparative tests were performed to compare the liquefied tissue scopes of the treatment probe of the present invention and the electrolytic treatment probe in the prior art. As a result, the liquefied tissue scope of the treatment probe of the present invention was 17-20mm, and that of the electrolytic treatment probe in the prior art was less than or equal to 10mm. After the treatment, the situations at the bladder outlet of the patients were observed by fiber-cystoscope respectively, the results of which are the same to the in vitro results.

### Advantages

1. Through in vitro and in vivo and clinical trials, it has been shown that, compared to the electrolytic treatment probe in the prior art, as to the treatment probe of the present invention, the liquefied scope significantly enlarged, tissues were liquefying necrotized thoroughly, and there was no damage to the non-target organs and there was no recurrence after cure;
2. During the treatment, current fluctuations are small, no phenomenon of stopping treatment would occur due to the open circuit, so that the defects of large current fluctuation and open circuit in the treatment of the electrolytic treatment probe can be effectively eliminated;
3. After such treatment, the recovery time for the patients to urinate on their own is reduced byl-2 weeks, the maximum urinary flow rate and efficacy improve significantly;
4. During the treatment, due to the present probe liquefies and necrotizes the surrounding prostate tissue to show a columnar shape, with the increase of the electrical quantity, the therapeutic scope extends outward gradually, starting from the near-electrode area, until reaching a large therapeutic scope at the aimed therapeutic electrical quantity. Although solidified necrotic tissues are also produced in the anode area at the bladder outlet, such situation of tissue adhesion to the electrode would not be produced due to the dissolution into intravesical fluid and the encapsulation by the liquefied necrotic tissues from the cathode, so that the probe can be taken out immediately after the treatment, and the treatment probe is not necessarily remained in the body, as a result, the patients recover fast;
5. It is a preferred, safe and reliable treatment for the elderly high-risk patients who are not appropriate for surgery, so that the patient can obtain an early recovery;
6. It can relieve the financial burden of patients, and significantly reduce the clinical costs of treatment. Compared to various surgical treatment, 2/3 treatment costs can be saved, with no need to be hospitalized, the burden of the patients' family can be relieved, and the medical resources can be saved.

The invention is set out in the appended claims.

## Claims

1. A probe for treating prostate tissue by electrolytic liquefying, comprising a probe pipe body (10), a guiding head (9), and a positioning balloon (8); a drainage channel (2-1), a water-injection channel (1-1), a gas-injection channel (3-1) and a cathode channel (5) are arranged in the probe pipe body (10) along the axial direction; the guiding head (9) is disposed at the front end of the probe pipe body (10), the positioning balloon (8) is arranged behind the guiding head (9); a cathode electrode (7) is tightly wound around the outer surface of the probe pipe body (10) on the rear side of the positioning balloon (8), a leading-out end of the cathode electrode (7) is connected with an cathode plug (A) through the cathode channel (5); an anode electrode (6) is arranged between the positioning balloon (8) and the cathode electrode (7), is wound around the outer surface of the probe pipe body (10) by at least one circle, and is kept at a distance from the cathode electrode (7), **characterized in that** the anode electrode is arranged in a position that is closer to the positioning balloon (8) than to the cathode electrode (7) and a leading-out end of the anode electrode (6) is connected with an anode plug (B) through the drainage channel (2-1).

2. The probe according to claim 1, **characterized in that** the spacing between the anode electrode (6) and the cathode electrode (7) is 3-5 mm

3. The probe according to claim 1, **characterized in that** the anode electrode (6) is made of platinum-rhodium alloy wire with a diameter of 0.3mm.

4. The probe according to claim 1, **characterized in that** a bracing wire channel (4) is arranged within the effective length range in said probe pipe body (10) along the axial direction, and a flexible bracing wire is placed in the bracing wire channel (4) with its both ends fixed and connected to the pipe body.

5. The probe according to claim 1, **characterized in that** the cathode electrode (7) is intermittently arranged along the axial direction and is composed of several segments, the spacing between the segments is 5mm, the length of one of the segments is 5-10mm, and the length of each of the others is 5mm.

## Patentansprüche

1. Sonde zur Behandlung von Prostatagewebe durch elektrolytische Verflüssigung, umfassend einen Sondenrohrkörper (10), einen Führungskopf (9) und einen Positionierungsballon (8); wobei ein Entwässerungskanal (2-1), ein Wasserinjektionskanal (1-1), ein Gasinjektionskanal (3-1) und ein Kathodenkanal (5) in dem Sondenrohrkörper (10) entlang der Axialrichtung angeordnet sind; der Führungskopf (9) am vorderen Ende des Sondenrohrkörpers (10) angeordnet ist, der Positionierungsballon (8) hinter dem Führungskopf (9) angeordnet ist; eine Kathodenelektrode (7) um die Außenfläche des Sondenrohrkörpers (10) an der Rückseite des Positionierungsballons (8) eng gewikkelt ist, ein herausführendes Ende der Kathodenelektrode (7) mit einem Kathodenstecker (A) durch den Kathodenkanal (5) verbunden ist; eine Anodenelektrode (6) zwischen dem Positionierungsballon (8) und der Kathodenelektrode (7) angeordnet ist, um die Außenfläche des Sondenrohrkörpers (10) herum um zumindest einen Kreis gewickelt ist und in einem Abstand zur Kathodenelektrode (7) gehalten ist, **dadurch gekennzeichnet, dass** die Anodenelektrode in einer Position angeordnet ist, die sich näher an dem Positionierungsballon (8) als der Kathodenelektrode (7) angeordnet ist, und ein herausführendes Ende der Anodenelektrode (6) mit einem Anodenstecker (B) durch den Entwässerungskanal (2-1) verbunden ist.

2. Sonde nach Anspruch 1, **dadurch gekennzeichnet, dass** der Abstand zwischen der Anodenelektrode (6) und der Kathodenelektrode (7) 3-5 mm beträgt.

3. Sonde nach Anspruch 1, **dadurch gekennzeichnet, dass** die Anodenelektrode (6) aus einem Platinum-Rhodium-Legierungsdraht mit einem Durchmesser von 0,3 mm hergestellt ist.

4. Sonde nach Anspruch 1, **dadurch gekennzeichnet, dass** ein Spanndrahtkanal (4) innerhalb eines effektiven Längenbereichs in dem Sondenrohrkörper (10) entlang der Axialrichtung angeordnet ist, und ein flexibler Spanndraht in dem Spanndrahtkanal (4) angeordnet ist, dessen beiden Enden an dem Rohrkörper befestigt und mit diesem verbunden sind.

5. Sonde nach Anspruch 1, **dadurch gekennzeichnet, dass** die Kathodenelektrode (7) entlang der Axialrichtung intermittierend angeordnet ist und aus mehreren Segmenten zusammengesetzt ist, wobei der Abstand zwischen den Segmenten 5 mm, die Länge eines der Segmente 5-10 mm und die Länge von jeweils einem der anderen 5 mm beträgt.

## Revendications

1. Sonde de traitement du tissu prostatique par la liquéfaction électrolytique, comprenant un corps de sonde tubulaire (10), une tête de guidage (9), et un ballon de positionnement (8); un canal de drainage (2-1), un canal d'injection d'eau (1-1), un canal d'injection de gaz (3-1) et un canal de cathode (5) étant disposés dans le corps de sonde tubulaire (10) le long de la direction axiale; la tête de guidage (9) étant disposée à l'extrémité avant du corps de sonde tubulaire (10), le ballon de positionnement (8) étant arrangé derrière la tête de guidage (9); une électrode de cathode (7) étant enroulée de manière serrée autour de la surface extérieure du corps de sonde tubulaire (10) sur le côté arrière du ballon de positionnement (8), une extrémité de sortie de l'électrode de cathode (7) étant reliée à un bouchon de cathode (A) par l'intermédiaire du canal de cathode (5); une électrode d'anode (6) étant disposée entre le ballon de positionnement (8) et l'électrode de cathode (7), étant enroulée autour de la surface extérieure du corps de sonde tubulaire (10) par au moins un cercle, et étant maintenue à une distance de l'électrode de cathode (7), **caractérisée en ce que** l'électrode d'anode est arrangée dans une position qui est plus près du ballon de positionnement (8) que l'électrode de cathode (7) et une extrémité de sortie de l'électrode d'anode (6) est reliée avec un bouchon d'anode (B) par l'intermédiaire du canal de drainage (2-1).

2. Sonde selon la revendication 1, **caractérisée en ce que** l'espacement entre l'électrode d'anode (6) et l'électrode de cathode (7) est compris entre 3 et 5 mm.

3. Sonde selon la revendication 1, **caractérisée en ce que** l'électrode d'anode (6) est faite d'un fil d'alliage platine-rhodium ayant un diamètre de 0,3 mm.

4. Sonde selon la revendication 1, **caractérisée en ce qu'**un canal de fil tendeur (4) est disposé à l'intérieur de la plage de longueur effective dans ledit corps de sonde tubulaire (10) le long de la direction axiale, et un fil tendeur souple est placé dans le canal de fil tendeur (4) avec ses deux extrémités fixées et reliées au corps de sonde.

5. Sonde selon la revendication 1, **caractérisée en ce que** l'électrode de cathode (7) est par intermittence disposée le long de la direction axiale et se compose de plusieurs segments, l'espacement entre les segments est 5 mm, la longueur de l'un des segments est comprise entre 5 et 10 mm, et la longueur de chacun des autres est 5 mm.
